# EUROPEAN PATENT APPLICATION

(11) **EP 2 000 092 A2**
(43) Date of publication of application: **10.12.2008**
(21) Application number: 07713938.4
(22) Date of filing: 08.02.2007
(51) Int. Cl.: A61B 6/00, A61B 5/00, G03B 42/02, G06Q 50/00

(54) **SMALL-SCALE DIAGNOSTIC SYSTEM**

(30) Priority: 27.03.2006 JP 2006085670; 27.03.2006 JP 2006085824
(71) Applicant: Konica Minolta Medical & Graphic, Inc., Hino-shi, Tokyo 191-8511 (JP)
(72) Inventor: OKUZAWA, Jiro, Tokyo 1630512 (JP); MOTOKI, Wataru, Tokyo 1630512 (JP); UMEKI, Mamoru, Tokyo 1630512 (JP)
(74) Representative: Henkel, Feiler & Hänzel
(86) International application number: PCT/JP2007/052222
(87) International publication number: WO 2007/111047

(57) **Abstract**

A diagnostic system which provides the imaging operator with the situation of the patients staying in a small-scale facility such as a medical practitioner or a clinic without increasing the man-hour such as input of examination order information and improves the diagnosis efficiency. When a visiting patient is received and registered by and in a reception computer (5), a patient information list showing the patients who visited the facility on the day is created. A patient list screen is displayed on a display section (221) of a reading apparatus (202). On the patient list screen, patient information on the patient to be imaged is specified, the patient information on the specified patient is sent to a controller (3). When image data created by an image generating apparatus (2) is sent to the controller (3), the controller (3) associates the image data with the patient information received from the reading apparatus (202) and stores them in a server (4).

## Description

### TECHNICAL FIELD

The present invention relates to a diagnostic system and more particularly to a small-scale diagnostic system mainly used in a small-scale medical installation.

### BACKGROUND

Conventionally, a diagnostic system is known that when a patient visits the hospital, a technician radiographs the patient to be inspected using an image generating apparatus such as a CR (computed radiography) apparatus or an FPD (flat panel detector), and an image process such as a gradation process is added so as to make the obtained image useable for diagnosis, and the image-processed image is outputted to a doctor for video check.

In such a diagnostic system, a plurality of persons in charge such as a person in charge (a receptionist) of receiving a patient visited and issuing an inspection order, a person in charge (a technician) of radiographing actually the patient in the radiographing room and generating image data, a person in charge (a technician appointed from general technicians) of judging possibility of offer of the gradated image to diagnosis and when applicable, correcting the contrast and density, and a person in charge of video check (a doctor) of judging (diagnosing) existence of a disease on the basis of the image play their roles, thus the diagnosis progresses.

And, in a large-scale medical installation in which the conventional diagnostic system is expected to be applied (hereinafter, referred to as a large-scale installation), there are a plurality of image generating apparatuses and technicians for operating them and consoles for operating the image generating apparatuses and viewers by which doctors confirm image data are installed separately so as to respectively play roles. Accordingly, there is a fear that a patient and image data may be mistaken. Therefore, to prevent it, a system for interconnecting each apparatus via a network, issuing an ID in each apparatus, and relating results of the operation process performed by each apparatus to each other is proposed (for example, refer to Patent Document 1).

In such a system, the places for playing the roles aforementioned are often separated from each other in a wide hospital such that the reception is arranged on the first floor and the radiation department is arranged underground, and in the radiation department, the case that a plurality of technicians execute simultaneously radiography for a plurality of patients using a plurality of radiographic apparatuses is stationary, and a plurality of patients stay always at each process, and to prevent a formed image and each patient from a wrong correspondence, an ID is assigned to each operation at each process, and they are brought into association with each other via a network of an HIS (hospital information system) or an RIS (radiology information system) (for example, refer to Patent Documents 2 and 3).

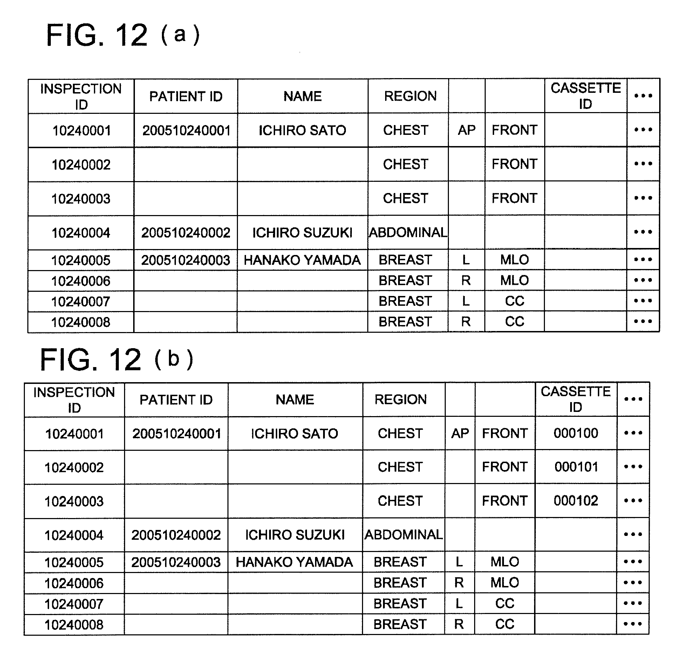

For example, the reception on the first floor decides the inspection contents (radiographic contents) on the basis of the main complaint of a patient and enters the contents together with the patient name. By doing this, a patient list as shown in Fig. 12(a) is formed. The patient list is added whenever necessary and is displayed on the work station for the reception on the first floor. Simultaneously, the patient list, via the network of the RIS or HIS, is displayed on the console (here, the console is referred to as a workstation installed in the radiation department for displaying the setting of radiographing conditions, the radiographing order information of the HIS, and a radiographed image of a patient) of the radiation department in the first basement. Further, the number of consoles installed, to increase the dispersion efficiency, is always more than one, though the consoles are also connected mutually via the network and when a predetermined inspection ID is selected by an optional console, to prevent duplicated radiography between a plurality of technicians, a method for notifying the purport of under processing in the concerned patient list (the flashing display or color is changed or if the same inspection is designated, a warning beep sounds) is used.

A technician of the radiation department uses the console close to himself, selects the inspection ID to be radiographed hereafter from the patient list displayed, and enters the ID (cassette ID) of the CR plate (cassette) used. By doing this, as shown in Fig. 12(b), the cassette ID entered in the field of "Cassette ID" of the patient list is displayed. The technician moves to the radiographing room, for example, with three cassettes and radiographs the patient. Thereafter, he reads the photographed cassette by the reading apparatus. The reading apparatus reads the cassette ID attached to the inserted cassette, attaches it to the image data, and transmits the concerned cassette ID, thus finally, the inspection ID (patient ID) is brought into association with the generated image data. The generated image data is transmitted to the console from which the technician selects the inspection ID and is displayed on the console. At this stage, he confirms the radiography positioning, when the positioning is defective, executes again radiography, and judges whether the correction of density and contrast and the frequency emphasizing processing are to be applied or not. Thereafter, he retains the concerned image in the video check waiting (diagnosis waiting) server. The video check doctor extracts and displays the image relating to the predetermined patient from the images preserved in the workstation of the video check room (often having a high-resolution monitor for the viewer function) and the aforementioned video check waiting server and then executes the video check (diagnosis).

And, in a system used in such a large-scale installation, with respect to the information influencing calculation of insurance points such that, for example, the radiography executed for a patient is simple radiography or contrast medium radiography, by entering the inspection ID and cassette ID of the patient and bringing the patient into association with the radiographic image, all the information is interconnected, is summarized in the RIS or HIS server, thereby can be managed.
Patent Document 1: U.S. Patent 5334851
Patent Document 2: Japanese Unexamined Patent Application Publication No. 2002-159476
Patent Document 3: Japanese Unexamined Patent Application Publication No. 2002-311524

### SUMMARY OF THE INVENTION

### PROBLEMS TO BE SOLVED BY THE INVENTION

However, according to the investigation of the inventors of the patent application, in a comparatively small-scale medical installation (hereinafter, referred to as a small-scale installation) such as a medical practioner or a clinic, there are found many cases in which there are very few image generating apparatuses installed, and an assistant positions a patient, and a doctor notified of completion of positioning from the assistant controls the X-ray irradiation switch or a doctor himself performs all operations including positioning of a patient.

Further, for example, in a large-scale installation, during the period from radiographing to diagnosis by a doctor, it may be supposed that a patient must move back and forth on many floors in the installation, while in a small-scale installation, the installation is narrow, so that during the period from radiographing to diagnosis by a doctor, the movement distance of a patient is short.

Under such conditions, it may be hardly considered that the radiographic image is mistaken for the patient and if the similar system to that of a large-scale installation is used, an operation of generating inspection order information including input of the patient name is necessary and on the contrary, the procedure is complicated, and the diagnostic efficiency is low.

Further, to generate beforehand inspection order information of a patient such as patient information and inspection information and bring the concerned inspection order information into association with the radiographic image, a system for connecting the apparatuses to each other with a network corresponding to a basic system such as the HIS or RIS is necessary, though a problem arises that the construction of such a system is expensive and it imposes a burden on a small-scale installation. Further, even if the numbers of respective apparatuses are decreased straight in the constitution concept of the aforementioned large-scale installation, it cannot be said that it is optimum to a small-scale installation.

On the other hand, if the patient is not brought into association with the radiographic image, for example, when the inspection is executed again for the same patient, the radiographic images of the concerned patient who was radiographed in the past cannot be sought out, and they cannot be used as a comparison image when judging the recovery status, so that a problem arises that the radiographic image cannot be used effectively.

Further, even in a small-scale installation such as a medical practitioner or a clinic, it is important for improving the diagnostic efficiency that a radiography executor such as a doctor for executing radiography or a radiographing technician can confirm the situation of a patient who stays in the installation and may be radiographed.

Therefore, the present invention was developed to solve the aforementioned problem and is intended to provide a diagnostic system, in a small-scale installation such as a medical practitioner or a clinic, without increasing the work man-hour such as input of the inspection order information, for offering the situation of a patient who stays in the installation and may be radiographed to a radiography executor and improving the diagnostic efficiency.

### MEANS FOR SOLVING THE PROBLEMS

To solve the aforementioned problem, the small-scale diagnostic system of the invention stated in Claim 1 comprises: a reception registration section to receive and register patient information of a visited patient; a list generation section to generate a list of the patient information which is received and registered by the reception registration section; and an image generating apparatus adapted to generate radiographic image data of an subject region of a patient to be inspected, wherein the image generating apparatus has a display section adapted to display the list of the patient information generated by the list generation section.

The invention stated in Claim 2, in the invention stated in Claim 1, is characterized in that it comprises: a patient designation section adapted to input a designation of patient information on a patient to be radiographed among the patient information included in the list displayed on the display section; an association section to bring the patient information designated by the patient designation section into association with the radiographic image data formed by the image generating apparatus during a period from a designation of the patient information by the patient designation section to a designation of next patient information; and a storing section to store the radiographic image data and patient information which are brought into association by the association section.

The invention stated in Claim 3, in the invention stated in Claim 1 or 2, is characterized in that the reception registration section is installed in a computer having a reception information generation section for generating reception related information on the registered patient relating to the accounts and reception.

The invention stated in Claim 4, in the invention stated in any one of Claims 1 to 3, is characterized in that the list generation section generates a list including at least an order of the reception and registration and patient information, and that the list including at least an order of the reception and registration and patient information is displayed on the display section.

The invention stated in Claim 5, in the invention stated in any one of Claims 1 to 4, is characterized in that the list generation section, when the patient information is received and registered by the reception registration section or when the generation of reception related information is finished by the reception information generation section, updates the list.

The invention stated in Claim 6, in the invention stated in any one of Claims 1 to 5, is characterized in that the image generating apparatus is a radiation image reading apparatus.

The invention stated in Claim 7, in the invention stated in Claim 2, further comprising: a reception information generation section for generating reception related information on the registered patient relating to the accounts and reception, wherein the image generating apparatus is a radiation image reading apparatus which reads radiation image information of a part to be inspected of the patient from a cassette, in which a photostimulable phosphor plate is built-in, used for radiographing of the part to be inspected, the radiation image information is recorded in a photostimulable phosphor plate and generates image data, wherein the radiation image reading apparatus comprises: a detection section to detect mounting of the cassette in the radiation image reading apparatus; a display section; and a notification section, when patient information of a patient to be radiographed is designated by the patient designation section and mounting of the cassette is detected by the detection section, to notify the patient information designated by the patient designation section and the cassette mounting information to the reception information generation section, wherein the reception information generation section, on the basis of the patient information and cassette mounting information notified from the radiation image reading apparatus, generates reception related information relating to the patient corresponding to the notified patient information.

The invention stated in Claim 8, in the invention stated in Claim 7, is characterized in that the cassette is attached with cassette information relating to the cassette, and the radiation image reading apparatus has a cassette information acquisition section for acquiring the cassette information of the cassette mounted in the radiation image reading apparatus, and the notification section notifies the patient information and the cassette mounting information and also the cassette information acquired by the cassette information acquisition section to the reception information generation section.

The invention stated in Claim 9, in the invention stated in Claim 8, is characterized in that the cassette information is cassette size information.

The invention stated in Claim 10, in the invention stated in Claim 8, is characterized in that the cassette information is classification information of the photostimulable phosphor plate built in the cassette.

### EFFECTS OF THE INVENTION

According to the invention stated in Claim 1, using patient information entered by the reception when he visits the hospital, a patient list for radiography is formed automatically and is displayed on the display section of the image generating apparatus. By doing this, to a radiography executor in the radiographing room, the patient information to be radiographed can be provided.

In this case, in a small-scale installation, a doctor grasping the radiographic contents is mostly a radiography executor, so that there is no need to positively prepare inspection order information. Therefore, there is no need to perform the input operation of the inspection order information by a man in full service which is executed in a conventional large hospital system. Further, a doctor for performing medical examination does not need to perform an unfamiliar input operation by the keyboard. As a result, the diagnostic efficiency will not be lowered.

Further, the radiography executor, in the radiographing room, can confirm the situation of a patient staying in the installation who may be radiographed, thus the diagnostic efficiency can be improved.

According to the invention stated in Claim 2, the patient information designated from the patient information list displayed on the display section of the image generating apparatus is automatically brought into association with the radiographic image data radiographed before the next patient information is designated, so that the patient can be brought correctly into association with the radiographic image data, and the physical and spiritual burden imposed on the doctor for preventing the patient and radiographic image data from being mistaken can be lightened, and the diagnostic efficiency can be improved more.

According to the invention stated in Claim 3, the patient information list can be formed using the computer for generating the reception related information, so that there is no need to provide a special operator as in a large-scale installation and labor saving can be realized.

According to the invention stated in Claim 4, the radiography executor can confirm the reception and entering procedure and patient information of a patient staying in the installation.

According to the invention stated in Claim 5, the patient information list is updated in association with a visit of a patient to the hospital and leaving it, so that the radiography executor, in the radiographing room, can confirm the update situation of a patient who stays in the installation and may be radiographed, thus the diagnostic efficiency can be improved.

According to the invention stated in Claim 7, if the cassette is mounted in the radiation image reading apparatus, the patient information to be radiographed and the cassette mounting information are notified to the reception information generation means, and the reception related information is notified automatically, so that an operator in charge of reception can save his labor of inputting the number of radiographs by viewing paper record cards, and the operation man-hour in the medical installation is reduced, and the processes executed after generation of the reception related information, for example, the processes of accounting and insurance point calculation can be executed earlier, thus the work flow in the installation can be made efficient.

According to the inventions stated in Claims 8 to 10, if the cassette is mounted in the radiation image reading apparatus, the patient information to be radiographed, cassette mounting information, and cassette information are notified to the reception information generation means, and the reception related information is notified automatically, so that an operator in charge of reception can save his labor of inputting the number of radiographs by viewing paper record cards and cassette information (for example, the cassette size, classification information of the photostimulable phosphor plate, etc.), and the operation man-hour in the medical installation is reduced, and the processes executed after generation of the reception related information, for example, the processes of accounting and insurance point calculation can be executed earlier, thus the work flow in the installation can be made efficient.

### BRIEF DESCRIPTION OF THE DRAWINGS

Fig. 1 is a drawing showing the entire constitution example of a small-scale diagnostic system 1 relating to the present invention.
Fig. 2 is a drawing showing an arrangement example of the apparatuses in a medical installation when the small-scale diagnostic system 1 shown in Fig. 1 is applied.
Fig. 3 is a block diagram of the essential section showing the functional constitution of a reading apparatus 202 applied to the small-scale diagnostic system 1 shown in Fig. 1.
Fig. 4 is a block diagram of the essential section showing the functional constitution of a controller 3 applied to the small-scale diagnostic system 1 shown in Fig. 1.
Fig. 5 is a block diagram of the essential section showing the functional constitution of a reception computer 5 applied to the small-scale diagnostic system 1 shown in Fig. 1.
Fig. 6 is a drawing showing a data storage example of a reception DB 57 shown in Fig. 1.
Fig. 7 is a flow chart showing the flow of the processes executed in the small-scale diagnostic system 1 during the period from a visit of a patient to the hospital to leaving it.
Fig. 8 is a drawing showing an example of a reception input screen 551 displayed on a display section 55 shown in Fig. 5.
Fig. 9 is a drawing showing an example of a patient list screen 223 displayed on a display section 221 shown in Fig. 3.
Fig. 10 is a drawing showing an example of a radiographic image display screen 351 displayed on a display section 35 shown in Fig. 4.
Fig. 11 is a drawing showing an example of the body part icon displayed on the display section 221 shown in Fig. 3.
Fig. 12(a) is a drawing showing an example of a list entered at the reception in a conventional diagnostic system and Fig. 12(b) is a drawing showing an example when a technician of the radiation department enters a cassette in the list shown in Fig. 12(a) in the conventional diagnostic system.

### DESCRIPTION OF NUMERALS

- 1: Small-scale diagnostic system
- 2: Image generating apparatus
- 2a: Ultrasonic diagnostic apparatus
- 20: Converter
- 2b: Endoscope apparatus
- 2c: CR apparatus
- 201: Radiographic apparatus
- 202: Reading apparatus
- 21: CPU
- 22: Operation-display section
- 221: Display section
- 222: Touch panel
- 223: Patient list screen
- 23: Communication section
- 24: RAM
- 25: Storing section
- 26: Image generation section
- 27: Bar code reader
- 28: Mounting sensor
- 29: Bus
- 3: Controller
- 31: CPU
- 32: RAM
- 33: Storing section
- 331: Temporary storing section
- 34: Input section
- 35: Display section
- 351: Radiographic image display screen
- 36: Communication section
- 37: Bus
- 4: Server
- 40: Image DB
- 5: Reception computer
- 51: CPU
- 52: RAM
- 53: Storing section
- 54: Input section
- 55: Display section
- 551: Reception input screen
- 56: Communication section
- 57: Reception DB
- 58: Bus
- 6: Network

### DESCRIPTION OF THE PREFERRED EMBODIMENTS

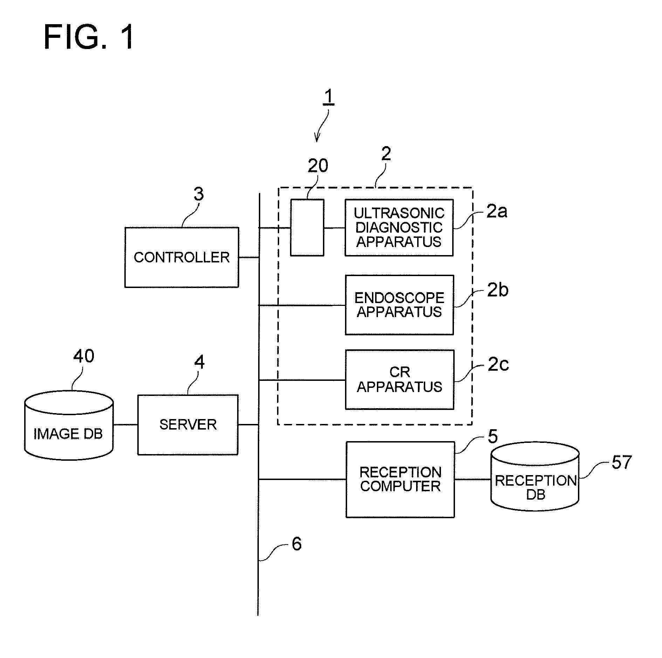

Hereinafter, an embodiment of the small-scale diagnostic system relating to the present invention will be explained with reference to Figs. 1 to 11. However, the present invention is not limited to the illustrations.

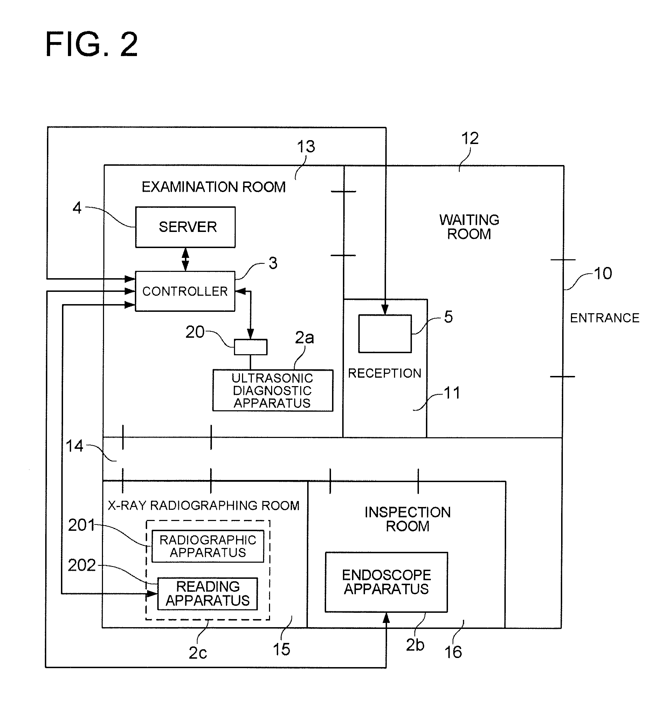

Fig. 1 shows the system constitution of the small-scale diagnostic system 1 of this embodiment and Fig. 2 shows an arrangement example of the apparatuses in a medical installation when the small-scale diagnostic system 1 is applied.

The small-scale diagnostic system 1 is a system to be applied to a small-scale installation such as a medical practioner or a clinic, and as shown in Fig. 1, it is composed of an ultrasonic diagnostic apparatus 2a which is an image generating apparatus 2, an endoscope apparatus 2b, a CR apparatus 2c, a controller 3, a server 4, and a reception computer 5, and each apparatus, for example, via a switching hub not drawn, is connected to a communication network (hereinafter, referred to as just a "network") 6 such as a LAN (local area network).

As a communication system in a hospital installation, generally, the DICOM (Digital Image and Communications in Medicine) standard is used and for communication between apparatuses connected to the LAN, the DICOM MWM (Modality Worklist Management) standard and DICOM MPPS (Modality Performed Procedure Step) standard are used. Further, the communication systems applicable to this embodiment are not limited to the aforementioned.

For example, a small-scale installation such as a medical practioner or a clinic, each apparatus is arranged as shown in Fig. 2.

Namely, when a patient enters an entrance 10, there are a reception 11 for receiving him and a waiting room 12. In the reception 11, a person in charge of reception is arranged and the person in charge gives a reception No. ticket on which the reception No. (the serial number in the today's reception order) for distinguishing each patient in the reception order is printed to each visited patient. In the reception 11, the reception computer 5 for executing insurance point calculation and accounting is installed and the person in charge of reception asks the name of a patient and inputs the correspondence of the reception No. to the patient name to the reception computer 5. Furthermore, the person in charge of reception, on the basis of the record card information after the end of diagnosis of the patient, performs an operation of inputting necessary information of the information relating to the accounts and reception (accounting and insurance point claim calculation) (hereinafter, referred to as reception related information) to the reception computer 5.

In the neighborhood of the waiting room 12, an examination room 13 where a doctor examines and diagnoses a patient is installed across the door. For example, on the examination desk (not drawn) in the examination room 13, the controller 3 used to display a radiographic image of the patient and make an image diagnosis by the doctor and the server 4 having an image DB (data base) 40 for storing image data of the radiographic image are arranged. In the examination room 13, the ultrasonic diagnostic apparatus 2a which is little necessary to be operated in an isolated zone from the viewpoint of privacy is installed.

Further, on the opposite side of the examination room 13 across a passageway 14, an X-ray radiographic room 15 for executing X-ray radiography is installed. In the X-ray radiographic room 15, the CR apparatus 2c composed of a radiographic apparatus 201 and a reading apparatus 202 is arranged. Furthermore, in the neighborhood of the X-ray radiographic room 15, an inspection room 16 is installed and the endoscope apparatus 2b is arranged in the inspection room 16.

Hereinafter, the small-scale diagnostic system 1 will be explained in detail.

Firstly, the constitution of each apparatus will be explained.

The image generating apparatus 2 is a modality, for example, for radiographing the inspected part of a patient as a subject by the ultrasonic diagnostic apparatus 2a, endoscope apparatus 2b, and CR apparatus 2c, converting the radiographed image to digital, thereby generating image data of the radiographic image.

The ultrasonic diagnostic apparatus 2a is composed of an ultrasonic probe for outputting ultrasonic waves and an electronic device connected to the ultrasonic probe for converting acoustic waves (an echo signal) received by the ultrasonic probe to image data of a radiographic image of the internal tissue (both are not drawn). The ultrasonic diagnostic apparatus 2a sends acoustic waves into the body from the ultrasonic probe, receives again the acoustic waves (an echo signal) reflected from the intra-body tissue by the ultrasonic probe, and generates a radiographic image corresponding to the echo signal by the electronic device.

To the ultrasonic diagnostic apparatus 2a, a converter 20 which is a converting means for converting an analog signal to a digital signal is connected and the ultrasonic diagnostic apparatus 2a is connected to a network 6 via the converter 20. Via the converter 20 like this, even when data of a form not conforming to the standard (for example, a communication protocol) of another external device connected to the network 6 is outputted from the ultrasonic diagnostic apparatus 2a, by appropriate conversion, data can be transmitted or received between itself and the external device connected to the network 6.

The endoscope apparatus 2b is an apparatus in which a small radiographic apparatus is installed at the leading edge of a flexible tube (both are not drawn) and the radiographic apparatus includes, for example, an objective optical system composed of an optical lens, a radiographing section arranged at the imaging position of the objective optical system, and a lighting section composed of an LED (light emitting diode) for lighting for radiographing (these are all not drawn). The radiographing section includes, for example, a solid-state radiographic device such as a CCD (charge coupled device) or a CMOS (complementary metal oxide semiconductor), which if light enters, converts photoelectrically it to an electric signal according to the incident light quantity of the light. The objective optical system is structured so as to collect light from the region lighted by the lighting section by the optical lens and form an image on the solid-state radiographic device of the radiographing section, and the light entering the solid-state radiographic device is converted photoelectrically, thus the image data of the radiographic image is outputted as an electric signal.

The CR apparatus 2c is an apparatus for executing radiography for a subject using a photostimulable phosphor plate, storing radiation energy transmitting the subject in the photostimulable phosphor plate, reading an image stored in the photostimulable phosphor plate, thereby generating image data of the radiographic image. In the CR apparatus 2c, the reading apparatus (the radiation image reading apparatus) has a radiation source and contains the photostimulable phosphor plate and there are a type of executing from radiographing to reading using one apparatus and a type using a portable cassette for storing a photostimulable phosphor plate available. In this embodiment, the cassette-type CR apparatus is illustrated, though the present invention is not limited to it.

The CR apparatus 2c is composed of the radiographic apparatus 201 having a radiation source and the reading apparatus 202 for reading an image from the photostimulable phosphor plate stored in the cassette used for radiation in the radiographic apparatus 201 and generating radiographic image data (refer to Fig. 2). Further, to the cassette, a bar code indicating the cassette information is attached. The cassette information is information indicating the attribute of the concerned cassette and concretely, it includes the cassette information indicating the size of the concerned cassette and the information (plate classification information) indicating the classification of the photostimulable phosphor plate built in the cassette. The plate classification information is information indicating whether it is a plate used for mammography radiography which is added to the insurance points or a plate used for radiography of the other parts.

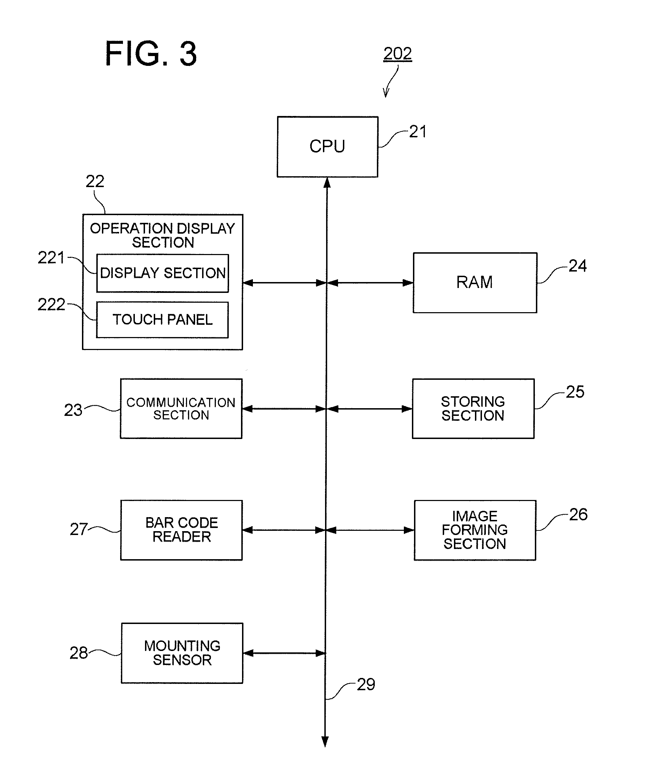

Fig. 3 is a block diagram showing the functional constitution of the reading apparatus 202. As shown in Fig. 3, the reading apparatus 202 includes a CPU 21, an operation display section 22, a communication section 23, a RAM 24, a storing section 25, an image generation section 26, a bar code reader 27, and a mounting sensor 28 and the respective sections are connected to each other with a bus 29.

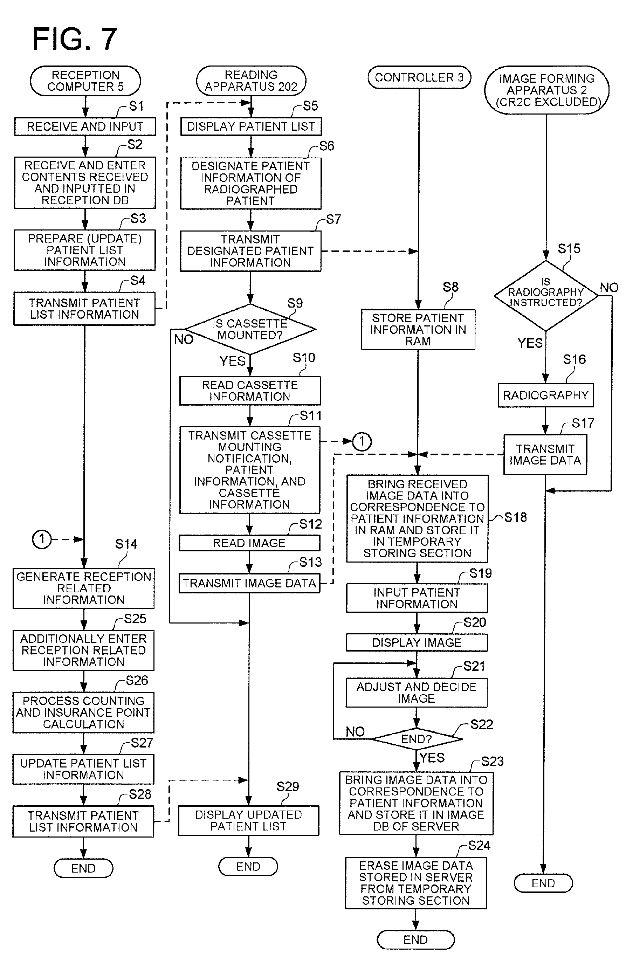

The CPU 21 reads the control program stored in the storing section 25, stores it in the work area formed in the RAM 24, and controls each section of the reading apparatus 202 according to the control program. Further, the CPU 21, according to the control program, reads various processing programs stored in the storing section 25, stores them in the work area, and in cooperation with the read programs, executes various processes including the process of the reading apparatus 202 shown in Fig. 7. The CPU 21 functions as a communication means of the present invention.

The operation display section 22 is composed of a display section 221 and a touch panel 222. The display section 221 is composed of a display screen composed of a LCD (liquid crystal display) and according to an instruction of a display signal inputted from the CPU 21, displays a patient list on the display screen.

The touch panel 222 is installed so as to cover the top of the display section 221, detects a desired input position inputted by an operation of a user using his finger, and outputs the detection signal to the CPU 21. The touch panel 222 functions as a patient designation means of the present invention.

The communication section 23 is composed of a network interface and transfers data with an external device connected to the network 6.

The RAM 24, in various processes executed and controlled by the CPU 21, forms a work area for temporarily storing various programs which are read from the storing section 25 and can be executed by the CPU 21, input or output data, and parameters.

The storing section 25 is composed of a nonvolatile semiconductor memory and stores the control program executed by the CPU 21, various programs, and various data.

The image generation section 26 is structured so as to mount the cassette used for radiography, fetches the photostimulable phosphor plate from the mounted cassette, scans it with exciting light, emits stimulated light from the radiation image information stored in the photostimulable phosphor plate, and on the basis of an image signal obtained by photoelectrically reading the stimulated light emitted, generates image data.

The bar code reader 27 reads the bar code displayed on the cassette and outputs it to the CPU 21.

The mounting sensor 28 detects existence of mounting of the cassette into the image generation section 26 and outputs the detection signal to the CPU 21.

The controller 3 is installed, for example, in the examination room 13, and it is a work station where the image data transmitted from the image generating apparatus 26 and patient information are brought into association with each other and a doctor displays an image and makes a video check diagnosis, and it may include a higher-resolution monitor than a monitor used in the general PC (personal computer).

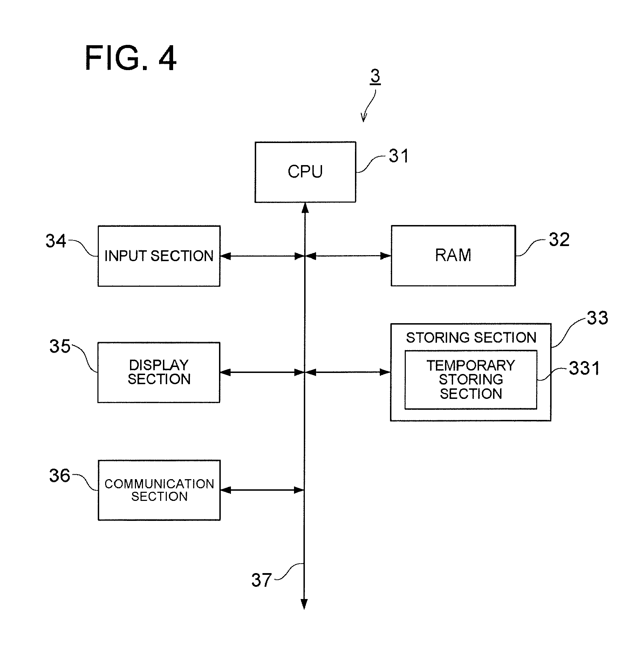

Fig. 4 is a block diagram showing the functional constitution of the controller 3. As shown in Fig. 4, the controller 3 is composed of a CPU 31, a RAM 32, a storing section 33, an input section 34, a display section 35, and a communication 36 and the respective sections are connected to each other with a bus 37.

The CPU 31 reads various programs such as the system program and processing programs stored in the storing section 33, stores them in the RAM 32, and according to the stored programs, executes various processes including the process of the controller 3 shown in Fig. 7. The CPU 31 functions as a corresponding means of the present invention.

The RAM 32, in various processes executed and controlled by the CPU 31, forms a work area for temporarily storing various programs which are read from the storing section 33 and can be executed by the CPU 31, input or output data, and parameters. For example, the RAM 32 forms a patient information region for storing temporarily patient information received from the reading apparatus 202.

The storing section 33 is composed of an HDD (hard disc) or a nonvolatile memory of a semiconductor. In the storing section 33, the system program executed by the CPU 31 and various programs are stored and as disclosed in the specifications of Japanese Unexamined Patent Application Publication No. 11-85950 and Japanese Unexamined Patent Application Publication No. 2001-76141, the part discrimination parameters (a lookup table for bringing the contour and shape to be radiographed appearing in a radiographic image into association with the radiographing part, etc.) for discriminating the radiographing part and the image processing parameters (a lookup table for defining the gradation curve used for the gradation process, the emphasizing degree of the frequency process, etc.) for performing the image processing according to the radiographing part discriminated are stored.

Further, the storing section 33 has a temporary storing section 331 for temporarily storing the image data transmitted from the image generating apparatus 2. The storing section 33 functions as a storing means of the present invention.

The input section 34 is composed of a keyboard including a cursor key, numeral input keys, and various function keys and a pointing device such as a mouse and outputs a pressing signal of a key operated from the keyboard and an operation signal by the mouse to the CPU 31 as an input signal.

The display section 35 is composed of, for example, monitors of a CRT (cathode ray tube) and an LCD (liquid crystal display) and according to an instruction of a display signal inputted from the CPU 31, displays various screens.

The communication section 36 is composed of a network interface and transfers data with an external device connected to the network 6 via a switching hub.

Again in Fig. 1, the server 4 is a computer composed of a storing section composed of a CPU, a RAM, and an HDD and a communication section for controlling communication with each apparatus connected to the network 6 (both are not drawn). The server 4 has an image DB 40 and by the software process in cooperation with the programs stored in the CPU and storing section, brings the image data of the radiographic image which is instructed to be written from the controller 3 via the communication section into association with the supplementary information thereof (the information including the patient information), and then stores them in the image DB 40 as an image storing means and in compliance with the request from the controller 3, searches the image DB 40, reads the image data according to the request and supplementary information thereof, and transmits them to the controller 3.

The reception computer 5 is a computer for receiving and entering a patient visiting the hospital and executing accounting and insurance point calculation.

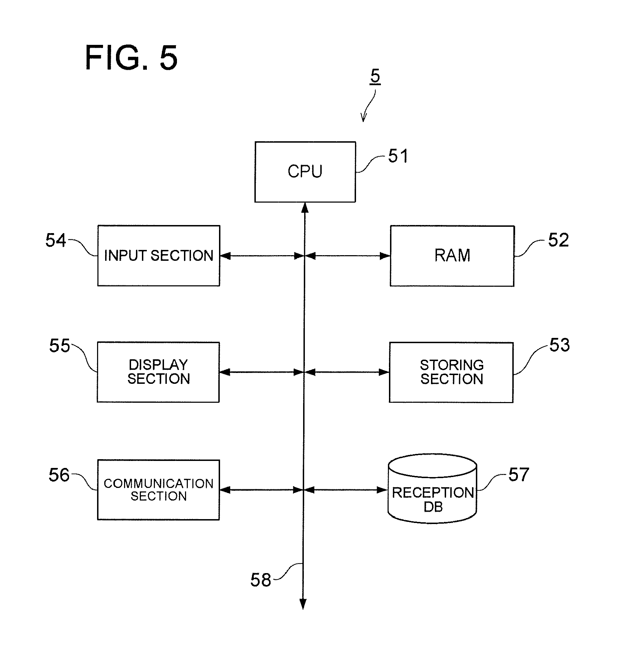

Fig. 5 is a block diagram showing the functional constitution of the reception computer 5. As shown in Fig. 5, the reception computer 5 is composed of a CPU 51, a RAM 52, a storing section 53, an input section 54, a display section 55, a communication section 56, and a reception DB 57 and the respective sections are connected to each other with a bus 58.

The CPU 51 reads various programs such as the system program and processing programs stored in the storing section 53, stores them in the RAM 52, and according to the stored programs, executes various processes including the process of the reception computer 5 shown in Fig. 7. The CPU 51 functions as a reception-entering means, a list forming means, and reception information generation means of the present invention.

The storing section 53 is composed of an HDD (hard disc) or a nonvolatile memory of a semiconductor and stores the system program executed by the CPU 51, various processing programs, and various data.

The input section 54 is composed of a keyboard including a cursor key, numeral input keys, and various function keys and a pointing device such as a mouse and outputs a pressing signal of a key operated from the keyboard and an operation signal by the mouse to the CPU 51 as an input signal.

The display section 55 is composed of, for example, monitors of a CRT and an LCD and according to an instruction of a display signal inputted from the CPU 51, displays various screens.

The communication section 56 is composed of a network interface and transfers data with an external device connected to the network 6 via a switching hub.

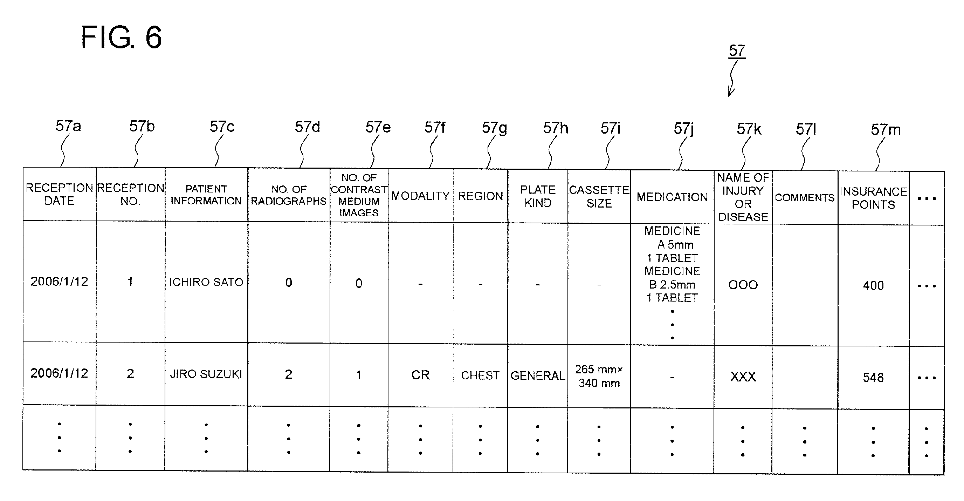

The reception DB 57 is a data base for bringing the reception related information relating to a visited patient into association with the patient information and storing it. Fig. 6 shows a data storage example of the reception DB 57. The reception DB 57 is a data base for storing reception related information of each patient visiting the hospital and as shown in Fig. 6, it includes a "Reception Date" item 57a for storing the reception data of a patient, a "Reception No." item 57b for storing the reception No. given to the patient, a "Patient Information" item 57c for storing the patient information (here, it is described as patient name), a "No. of radiographs" item 57d for storing the number of images radiographed for the patient on the reception date, a "No. of contrast medium images" item 57e for storing the number of radiographs using a contrast medium, a "Modality" item 57f for storing the kind of the image generating apparatus 2 executing radiography, a "Part" item 57g for storing the information on the radiographing part, a "Plate Kind" item 57h for storing the plate classification information used for radiography, a "Cassette Size" item 57i for storing the size information of the cassette used for radiography, a "Medication" item 57j for storing the medication information prescribed for the patient on the reception date, a "Name of Injury or Disease" item 57k for storing the name of injury or disease diagnosed by a doctor on the reception date, a "Comments" item 571 for storing comments inputted from the controller 3 which will be described later, and an "Insurance Points" item 57m for storing the insurance points calculated.

Next the operation of the small-scale diagnostic system will be explained.

Fig. 7 is a flow chart showing the flow of the processes executed in the small-scale diagnostic system 1 during the period from a visit of a patient to the hospital to leaving it. Hereinafter, by referring to Fig. 7, the flow of a series of processes in the small-scale diagnostic system during the period from a visit of a patient to the hospital to leaving it will be explained together with the work flow of the intra-installation staff (doctor, radiographing technician, person in charge of reception).

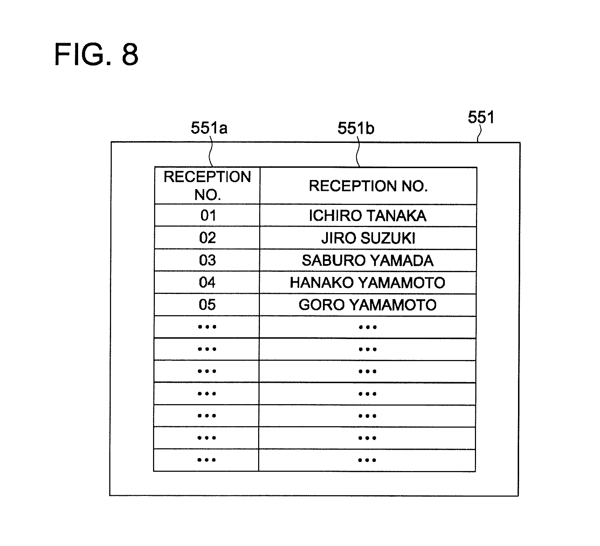

Firstly, at the reception 11, by the person in charge of reception, a reception No. ticket is given to the visited patient and the patient name is asked. Next, he operates the input section 54 of the reception computer 5 to display a reception input screen 551 (refer to Fig. 8), and the reception No. is input into a reception No. field 551a by the input section 54, and the patient name which is patient information is input into a patient name field 551b.

In the reception computer 5, if a display instruction of the reception input screen 551 is input from the input section 54, on the display section 55, the reception input screen 551 on which the reception No. field 551a and patient name field 551b for inputting the reception No. and patient information are provided is displayed, and if the patient information such as the reception No. and patient name is input into the reception No. field 551a and patient name field 551b by the input section 54 via the reception input screen 551 (Step S1), a new record is added to the reception DB 57, and the present date is written into the "Reception Date" item 57a, and the inputted reception No. is written into the "Reception No." item 57b, and the input patient information is written into the "Patient Information" item 57c, and they are received and entered (Step S2). Fig. 8 shows an example of the reception input screen 551.

In the reception computer 5, if the patient is received and entered, patient list information of the patient received and entered is generated (or updated) (Step S3). The patient list information, for example, is generated if the first patient is received and entered, is stored in a predetermined region of the RAM 52, and is updated to new patient list information whenever the next patient is received and entered. The patient list information, for example, if a record that the present date is stored in the "Reception Date" item 57a from the reception DB 57 and no data is stored in the items other than the "Reception Date" item 57a, the "Reception No." item 57b, and the "Patient Information" item 57c is extracted, is generated on the basis of the extracted record. The patient list information includes at least the patient information such as the reception No. indicating the reception order and patient name. The patient list information generated or updated is transmitted to the reading apparatus 202 via the communication section 56 (Step S4). In the reading apparatus 202, if the patient list information is received via the communication section 23, on the basis of the patient list information received, the patient list screen 223 is displayed on the display section 221 (Step S5).

The patient given the reception No. stands by in the waiting room 12 and then moves to the examination room 13. In the examination room 13, the doctor conducts a medical examination to the patient and the radiography (the kind of the image generating apparatus 2, radiographing part, radiographing direction, number of radiographs, etc.) to be executed for the concerned patient is decided. The patient, by an instruction of the radiography executor for executing radiography such as a doctor or a radiographing technician, moves in front of the image generating apparatus 2 (the ultrasonic diagnostic apparatus 2a, endoscope apparatus 2b, or CR apparatus 2c) for executing radiography.

The radiography executor, after the patient moves on the front of the image generating apparatus 2 for executing radiography, moves on the front of the reading apparatus 202 and designates the patient information to be radiographed from the patient list screen 223 displayed on the display section 221.

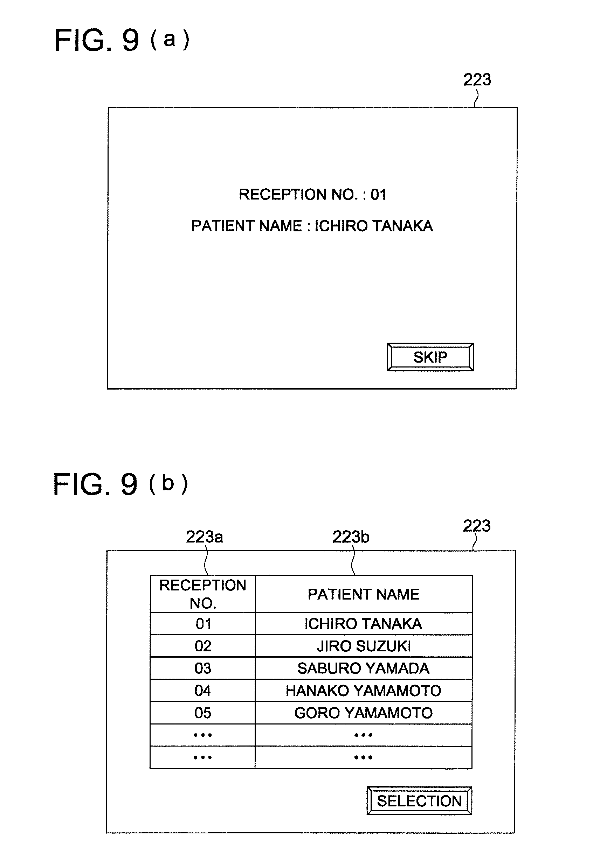

Figs. 9(a) and 9(b) show examples of the patient list screen 223. The patient list screen 223 displays the patient information list of a patient and receives designation of the patient information of the patient to be radiographed from the patient list.

Fig. 9(a) is a patient list screen as an aspect of displaying the reception No. and patient name of each patient from the top information of the patient list information. On the patient list screen 223, upon receipt of the patient list information, the top information is displayed first and if the "Skip" button is touched, the next information in the patient list information is displayed. If the screen is touched when the patient information of the patient to be radiographed is displayed, the displayed patient information is designated as patient information of the patient to be radiographed. The display of the patient list screen 223 shown in Fig. 9(a), when the screen of the display section 221 is small, can be changed so as to see easily the patient list.

Fig. 9(b) is a patient list screen including the reception No. field 223a and patient name field 223b as an aspect of listing the patient list information. It is selected by touching the patient information of the patient to be radiographed and if the "Selection" button is touched furthermore, the selected patient information is designated as patient information of the patient to be radiographed. The patient list screen 223 shown in Fig. 9(b), when the screen of the display section 221 is large, can display the patient list so as to be seen easily. Further, when the screen of the display section 221 is small, the display region of each patient information is small, so that the patient information can be hardly selected by the touch panel. Therefore, if a ten-key pad is installed separately instead of the touch panel 222, and the patient list is referred to, and a search ID (here, the reception No.) corresponding to the patient information designated as a radiographic subject is input via the ten-key pad, an aspect that the patient information corresponding the search ID is designated as patient information to be radiographed may be used.

If the list screen, as shown in Figs. 9(a) and 9(b), of a patient who is received and entered and does not leave yet the hospital, that is, a patient staying the installation is displayed on the reading apparatus 202 in the radiographing room 15, the radiography executor can easily confirm and grasp the update situation of the patient staying in the installation, and the diagnostic efficiency can be improved.

In the reading apparatus 202, if the patient information is designated from the patient list screen 223 by the touch panel 222 (Step S6), the designated patient information is transmitted to the controller 3 via the communication section 23 (Step S7). In the controller 3, upon receipt of the patient information from the reading apparatus 202, the received patient information is stored (overwritten and held) in the patient information region of the RAM 32 (Step S8).

If the designation of the patient information is finished, the radiography executor moves to the image generating apparatus 2 for executing radiography, radiographs the inspected part of the patient as a subject, and generates image of data of the radiographic image. When the image generating apparatus 2 for executing radiography is the CR apparatus 2c, the radiographic apparatus 201 executes radiography and mounts the radiographed cassette in the reading apparatus 202. In the reading apparatus 202, if the mounting of the radiographed cassette is detected by the mounting sensor 28 as a detection means (YES at Step S9), the bar code attached to the cassette is read by the bar code reader 27 as a cassette information acquisition means, and the cassette information is acquired (Step S10), and the cassette mounting notification, the patient information designated at present from the patient list screen, and the acquired cassette information are transmitted to the reception computer 5 via the communication section 23 (Step S11). Next, by the image generation section 26, the radiation image recorded on the mounted cassette is read and image data is generated (Step S12) and is transmitted to the controller 3 via the communication section 23 (Step S13). Further, it is preferable to supplement the patient information of the patient as a subject, that is, the patient information designated at Step S7 to the generated image data as supplementary information and transmit it to the controller 3. When a patient is radiographed several times under a plurality of radiographic conditions, Steps S9 to S13 are executed repeatedly.

In the reception computer 5, if the cassette mounting notification, patient information, and cassette information from the reading apparatus 202 are received by the communication section 56, on the basis of the received information, reception related information is generated (Step S14). Concretely, in the reception DB 57, the reception date is today, and a record having the received patient information is searched, and the "Number of Radiographs" item 57d of the record is counted up by 1, and the information indicating the CR apparatus is written into the "Modality" item 57f, and the plate classification information included in the cassette information is written into the "Plate Kind" item 57h, and the cassette size information included in the cassette information is written into the "Cassette Size" item 57i.

When the image generating apparatus 2 for executing radiography is other than the CR apparatus 2c, that is, when it is the ultrasonic diagnostic apparatus 2a or the endoscope apparatus 2b, if radiography is instructed from the input section (YES at Step S15), the radiography is executed by the radiography executor (Step S16) and the image data obtained by the radiography is transmitted to the controller 3 (Step S17). Further, when a patient is radiographed several times under a plurality of radiographic conditions, all the image data generated is transmitted to the controller 3.

In the controller 3, if image data is received from the reading apparatus 202 or the image generating apparatus 2, the received image data is brought into association with the patient information stored in the patient information region of the RAM 32 and is stored in the temporary storing section 331 (Step S18). Namely, in the controller 3, the patient information is designated by the reading apparatus 202 and during the period from reception of the designated patient information by the controller 3 to designation of the next patient information from the reading apparatus 202, the image data received from the reading apparatus 202 or the image generating apparatus 2 is brought into association with the patient information designated by the reading apparatus 202 and is stored. Further, if only one patient is always radiographed (when there are not a plurality of patients in the radiographing room), if the patient information is transmitted from the reading apparatus 202 to the controller 3, image data of the ultrasonic diagnostic apparatus 2a or endoscope apparatus 2b which is generated before the next patient information is transmitted can be brought into association with the patient information by the controller 3.

If the radiography is finished, the patient moves to the examination room 13. The doctor operates the input section 34 of the controller 3 to display an image searching screen not drawn on the display section 35 and inputs the patient information of the objective patient. The controller 3 displays the image searching screen according to the operation of the input section 34 and receives input of the patient information via the input section 34. If the image searching screen is displayed and the patient information is input from the screen via the input section 34 (Step S19), the image data corresponding to the input patient is extracted from the temporary storing section 331, and the image process such as the gradation process or frequency emphasizing process is performed for the extracted image data, and a thumbnail image which is image-processed image data contracted is prepared and is displayed on a radiographic image display screen 351 of the display section 35 (Step S20).

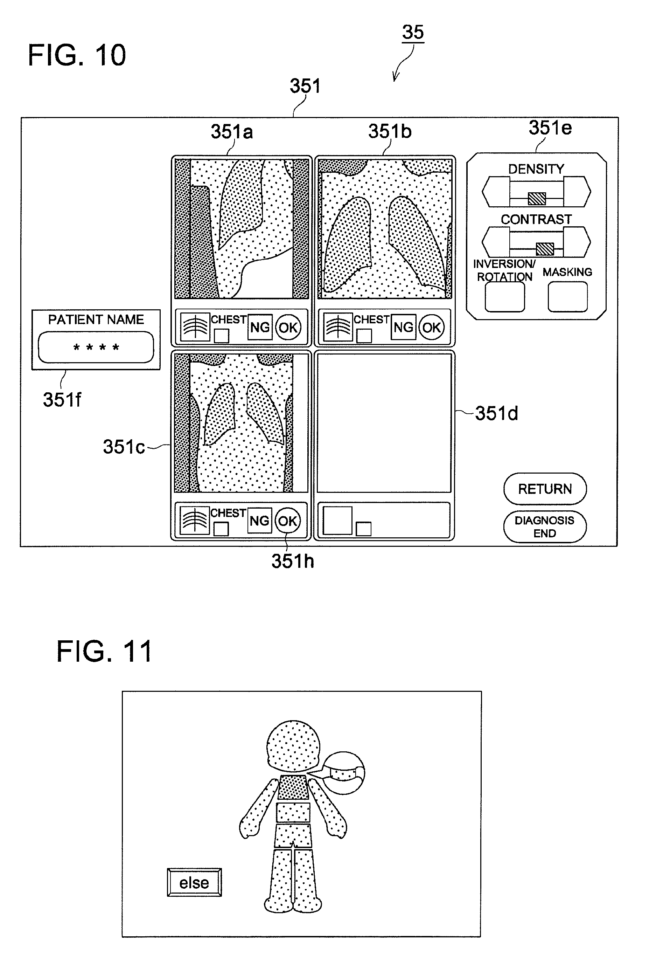

Fig. 10 shows a display screen example of the radiographic image display screen 351 displayed on the display section 35. As shown in Fig. 10, the radiographic image display screen 351 has image display fields 351a to 351d for listing the extracted images. If any of the image display fields 351a to 351d is selected by the mouse of the input section 34, the selected image is displayed independently in the life size. The doctor, from the independently displayed image, observes the image in detail and can make a video check diagnosis. Further, on the upper right of the screen, an image process adjustment field 351e is provided, and the doctor operates the image process adjustment field 351e by the mouse, thereby can adjust the density and contrast. If an OK button 351h displayed in correspondence with each image is pressed, the displayed image can be decided as an image to be stored in the image DB 40. Further, on the radiographic image display screen 351, a patient information display field 351f for displaying the patient information corresponding to the displayed radiographic image is provided.

After the image process adjustment and image decision are executed from the radiographic image display screen 351 via the input section 34 (Step S21), if the End button of the radiographic image display screen 351 is pressed by the input section 34 and the end of diagnosis is instructed (YES at Step S22), the image data corresponding the patient information of the concerned patient is transmitted to the server 4 via the communication section 36 and is stored in the image DB 40 (Step S23). The image data written into the image DB 40 of the server 4 is erased from the temporary storing section 331 (Step S24).

If the examination of the patient is finished in the examination room, the patient moves to the reception 11 and the account is settled. The doctor records his opinion (the name of injury or disease examined) on the concerned patient, the medication information indicating the medicine prescribed for the patient, and the information relating to the radiography executed for the patient (the apparatus kind executing radiography, the number of radiographs, existence of a contrast medium, the radiographing part, the radiographing direction, etc.) on a paper record card. And, he sends the paper record card to the reception 11.

The person in charge of reception displays the reception related information input screen (not drawn) on the reception computer 5 and from the reception related information input screen, on the basis of the description of the paper record card, inputs the reception No. and reception related information of the objective patient. Here, when radiography is executed by the CR apparatus 2c, the modality classification, the number of radiographs by the CR apparatus 2c, the plate kind, and the cassette size information are entered already, and the person in charge of reception does not need to input these information, so that the input operation is simplified and the account can be settled earlier.

On the reception computer 5, the information inputted from the reception related information input screen is added, entered, and stored in the corresponding item of the record of the reception DB 57 having the reception No. of the objective patient (Step S25). Further, on the basis of the input reception related information, the accounting information-insurance point calculation process for the patient is performed (Step S26). The person in charge of reception, on the basis of the calculated accounting information, charges the patient the fee for medical examination and settles the account. The patient pays the accounts and leaves the hospital.

On the reception computer 5, if the accounting information-insurance point calculation process is finished, it is judged that the patient leaves the hospital, and from the patient list information stored in the RAM 52, the reception related information is entered, and the patient information which is an object of accounting information-insurance point calculation is deleted and updated (Step S27), and the updated patient list information is transmitted to the reading apparatus 202 via the communication section 56 (Step S28). On the reading apparatus 202, for the next radiography, on the basis of the updated patient list information, the patient list screen is displayed (Step S29).

As explained above, according to the small-scale diagnostic system 1, on the reception computer 5, if visited patients are received and entered, a patient information list of the patients visited today is generated and the patient list screen is displayed on the display section 221 of the reading apparatus 202. If the patient information of the patient to be radiographed is designated from the patient list screen of the reading apparatus 202, the concerned designated patient information is transmitted to the controller 3 and is stored in the patient information region of the RAM 32 of the controller 3. In the image generating apparatus 2, if the patient is radiographed and image data is generated, the generated image data is transmitted to the controller 3. On the controller 3, if the image data is received, the image data is brought into association with the patient information stored in the patient information region of the RAM 32, is stored in the temporary storing section 331, is used for diagnosis of the doctor, and then is stored in the image DB 40 of the image server 4.

Therefore, using the information, on the reception computer 5, received and entered when a patient visits the hospital, patient list information for radiographing is generated automatically and is displayed on the display section 221 of the reading apparatus 202. By doing this, in the radiographing room, the patient information to be radiographed can be provided for the radiography executor.

In this case, in a small-scale installation, a doctor grasping the radiographic contents is mostly a radiography executor, so that there is no need to positively prepare inspection order information. Therefore, there is not need to perform the input operation of the inspection order information by a man in full service which is performed in a conventional large hospital system. Further, a doctor for performing medical examination does not need to perform an unfamiliar input operation by the keyboard. As a result, the diagnostic efficiency will not be lowered.

Further, the radiography executor, in the radiographing room, can confirm the update situation of a patient staying in the installation, thus the diagnostic efficiency can be improved.

Furthermore, if the patient information to be radiographed is designated from the patient list screen displayed on the display section 221, all the image data generated by the reading apparatus 202 and image generating apparatus 2 before the next different patient information is designated, in the controller 3, is automatically brought into association with the designated patient information, so that in the small-scale installation, the patient can be brought correctly into association with the image, and the physical and spiritual burden imposed on the doctor for preventing the patient and radiographic image from being mistaken can be lightened, and the diagnostic efficiency can be improved more.

Further, according to the small-scale diagnostic system 1, if the patient information of the patient to be radiographed is designated from the patient list screen of the reading apparatus 202 and the cassette is mounted, the bar code attached to the cassette is read, and the cassette information (the plate classification information and cassette size information) indicated by the bar code is acquired, and the acquired information is transmitted to the reception computer 5 together with the cassette mounting notification and designated patient information. In the reception computer 5, on the basis of the information received from the reading apparatus 202, plate related information for the designated patient is generated.

Therefore, at the time of radiography by the CR apparatus, the number of radiographs, plate classification information, and cassette size information which are necessary as reception related information are automatically notified the reception computer 5, and reception related information is generated automatically in the reception computer 5, so that the person in charge of reception can save his labor of looking at the paper record card and inputting these information, thus an input error is prevented, and the operation man-hour is reduced, and the processes executed after generation of the reception related information, for example, the processes of accounting and insurance point calculation can be executed earlier. As a result, the entire work flow in the installation can be made efficient.

Further, the description contents of the embodiment aforementioned are a preferable example of the small-scale diagnostic system 1 relating to the present invention and the present invention is not limited to it.

For example, in the aforementioned embodiment, on the reception computer 5, if the reception and leaving of the patient are entered, the patient list information is updated, and the updated patient list information is transmitted to the reading apparatus 202, thus the patient list screen of the patient staying at present in the installation is displayed on the reading apparatus 202, though when the patient is received and entered, the information relating to the added patient (for example, the reception No. and patient information, etc.) is transmitted from the reception computer 5 to the reading apparatus 202 and is added to the patient list screen of the display section 221, and when the patient is entered leaving the hospital, the information on the left patient (for example, the reception No. and patient information, etc.) is transmitted from the reception computer 5 to the reading apparatus 202 and may be deleted from the patient list screen of the display section 221. Further, a terminal used to input check-in and check-out by a patient is installed at the reception 11 and the input information (entering and leaving information) from the terminal is transmitted to the reading apparatus 202, and on the reading apparatus 202, the patient list screen of the patient staying at present in the installation may be displayed.

Further, the system, in correspondence with each patient information on the reception input screen, is structured so as to display a check field or an icon for designating a patient whose information is not added to the patient list, thus the person in charge of reception, when a patient visiting the hospital, for example, comes only to receive medicine or when he judges that no radiography is necessary from the latest name of injury or disease of the patient, at the time of reception and entering of the patient, may designate and input not to add him to the patient list, and the reception computer 5 may exclude the concerned designated patient information and generate a patient list. Further, it is possible to exclude the reception No. and display only the patient information as a patient list.

Further, in the aforementioned embodiment, the bar code indicating the cassette size information and the plate classification information showing whether it is mammographic radiography or not is attached to the cassette and is read by the bar code reader in the reading apparatus 202, thus the cassette size information and plate classification information are acquired, though in the reading apparatus 202, it is possible to analyze the generated image data, for example, generate a histogram of image data, and obtain the maximum and minimum values, thereby acquire the information on whether it is mammographic radiography or not.

Further, in the reading apparatus 202, when the cassette is mounted, it is possible to display, for example, a body part icon for selecting each part of the human body as shown in Fig. 11 on the display section 221, acquire the radiographing part information touched (selected) from the body part icon, and transmit the acquired radiographing part information to the reception computer 5 together with the cassette mounting notification, patient information, and cassette information. By doing this, the information on the radiographing part of the reception related information is generated automatically, and the person in charge of reception does not need to input it, and the work flow in the installation can be made more efficient.

Further, in the aforementioned embodiment, the CR apparatus is illustrated as a radiation image generating apparatus, though the FPD can be used as a radiation image generating apparatus. When using the FPD, disclosed in Japanese Unexamined Patent Application Publication No. 2000-131785, having a display section (including an external device) and an operation section, the patient list is displayed on the display section and the patient information to be radiographed can be selected from the patient list by the operation section. In this case, the selected patient information can be transmitted to the controller 3 or the reception computer 5 wirelessly or through wire.

Further, in the FPD, since it is expensive, the cassette is changed little according to the radiographing part unlike the CR cassette and a plurality of radiographic image data is stored generally in one cassette. At this time, the execution of radiography can be recognized on the basis of a trigger signal of the reading operation or reset operation, so that each of the plurality of radiographic image data can be stored with the patient information accompanied. Therefore, in the FPD, when transmitting the radiographic image data to the controller 3, it is preferable to transmit it with the patient information accompanied.

Further, with respect to the number of radiographs, whenever the radiographic image data is stored with the patient information accompanied or simultaneously with transmission to the controller 3, if the patient information is transmitted from the FPD to the reception computer 5 and the number of acquisition times of the same patient information is calculated by the reception computer 5, the number of radiographing times corresponding to the patient information can be discriminated, so that the number of radiographing times information can be used for precalculation of the insurance point process.

Furthermore, needless to say, the present invention is not limited to this embodiment and can be modified appropriately.

## Claims

1. A small-scale diagnostic system comprising:
a reception registration section to receive and register patient information of a visited patient;
a list generation section to generate a list of the patient information which is received and registered by the reception registration section; and
an image generating apparatus adapted to generate radiographic image data of an subject region of a patient to be inspected,
wherein the image generating apparatus has a display section adapted to display the list of the patient information generated by the list generation section.

2. The small-scale diagnostic system as described in claim 1, further comprising:
a patient designation section adapted to input a designation of patient information on a patient to be radiographed among the patient information included in the list displayed on the display section;
an association section to bring the patient information designated by the patient designation section into association with the radiographic image data formed by the image generating apparatus during a period from a designation of the patient information by the patient designation section to a designation of next patient information; and
a storing section to store the radiographic image data and patient information which are brought into association by the association section.

3. The small-scale diagnostic system as described in claim 1 or 2, wherein the reception registration section is installed in a computer having a reception information generation section for generating reception related information on the registered patient relating to the accounts and reception.

4. The small-scale diagnostic system as described in claims 1 to 3, wherein the list generation section generates a list including at least an order of the reception and registration and patient information, and wherein the list including at least an order of the reception and registration and patient information is displayed on the display section.

5. The small-scale diagnostic system as described in any one of claims 1 to 4, wherein the list generation section, when the patient information is received and registered by the reception registration section or when the generation of reception related information is finished by the reception information generation section, updates the list.

6. The small-scale diagnostic system as described in any one of claims 1 to 5, wherein the image generating apparatus is a radiation image reading apparatus.

7. The small-scale diagnostic system as described in claim 2, further comprising:
a reception information generation section for generating reception related information on the registered patient relating to the accounts and reception,
wherein the image generating apparatus is a radiation image reading apparatus which reads radiation image information of a part to be inspected of the patient from a cassette, in which a photostimulable phosphor plate is built-in, used for radiographing of the part to be inspected, the radiation image information is recorded in a photostimulable phosphor plate and generates image data,
wherein the radiation image reading apparatus comprises:
a detection section to detect mounting of the cassette in the radiation image reading apparatus;
a display section; and
a notification section, when patient information of a patient to be radiographed is designated by the patient designation section and mounting of the cassette is detected by the detection section, to notify the patient information designated by the patient designation section and the cassette mounting information to the reception information generation section,
wherein the reception information generation section, on the basis of the patient information and cassette mounting information notified from the radiation image reading apparatus, generates reception related information relating to the patient corresponding to the notified patient information.

8. The small-scale diagnostic system as described in claim 7, wherein, the cassette is attached with cassette information relating to the cassette, and the radiation image reading apparatus has a cassette information acquisition section for acquiring the cassette information of the cassette mounted in the radiation image reading apparatus, and the notification section notifies the patient information and the cassette mounting information and also the cassette information acquired by the cassette information acquisition section to the reception information generation section.

9. The small-scale diagnostic system as described in claim 8, wherein the cassette information is cassette size information.

10. The small-scale diagnostic system as described in claim 8, wherein the cassette information is classification information of the photostimulable phosphor plate built in the cassette.
